# EUROPEAN PATENT SPECIFICATION

(11) **EP 2 190 442 B1**
(45) Date of publication and mention of the grant of the patent: **10.10.2012**
(21) Application number: 08789829.2
(22) Date of filing: 25.08.2008
(51) Int. Cl.: A61N 1/05, A61N 1/20, A61K 33/38, A61B 17/58, A61B 17/34

(54) **AN APPARATUS FOR TREATMENT AND PREVENTION OF INFECTIOUS DISEASE**
GERÄT ZUR BEHANDLUNG UND PRÄVENTION VON INFEKTIONSKRANKHEITEN
DISPOSITIF POUR TRAITER ET PREVENIR UNE MALADIE INFECTIEUSE

(30) Priority: 30.08.2007 IL 18563707
(43) Date of publication of application: 02.06.2010
(73) Proprietor: Ortho-Ion Ltd., 36885 Nesher (IL)
(72) Inventor: SCHWARTZ, Liat, 34755 Haifa (IL); BARSKY, Valentin, 32697 Haifa (IL)
(74) Representative: Messulam, Alec Moses
(86) International application number: PCT/IL2008/001157
(87) International publication number: WO 2009/027968

(56) References cited:
- US-A- 3 784 908
- US-A- 4 313 438
- US-A- 4 314 554
- US-A- 4 886 075
- US-A- 5 395 398
- US-A- 6 149 620

## Description

### Field of the Invention

The invention is related to the field of medicine. Specifically the invention relates to the field of treatment and prevention of bacterial infections. More specifically the invention relates to bone and cartilage pathologies.

### Background of the Invention

Publications and other reference materials referred to herein, including reference cited therein, are incorporated herein by reference in their entirety and are numerically referenced in the following text and respectively grouped in the appended Bibliography which immediately precedes the claims.

In spite of very advanced diagnostic and treatment options, bone infection called Osteomyelitis remains one of the most serious complications in orthopedic surgery. On one hand it affects dramatically the quality of life of a patient and may risk his life. On the other hand it is a very expensive disease for the patient and society because of the involved costs of diagnosis, treatment, rehabilitation, lost of productivity and in many cases incomplete cure [8].

Osteomyelitis can affect all populations, infants, children, adults, and the elderly, while populations at increased risk include individuals with weakened immune system or other diseases increasing susceptibility to infections. It may be caused by a variety of microbial agents and situations, including [9]:
- An open injury to the bone, such as an open fracture;
- An infection from elsewhere in the body;
- A minor trauma, which can lead to a blood clot around the bone thus to reside in a secondary bone infection;
- Bacteria in the blood stream, which is deposited in a local area of the bone;
- A chronic open wound or soft tissue infection, that extends down to the bone.

If left untreated, the infection becomes chronic, and results in chronic Osteomyelitis (COM). COM is a major medical problem in most countries, mainly associated with violent trauma and orthopedic surgery. Its high impact is suffered equally in terms of increased morbidity, comprehensive use of health care resources and sequelae. In the opinion of authorized clinicians, the term cure cannot be applied to COM, because the bone infection may occur years after apparently successful treatment of the disease.

The objective of treating Osteomyelitis is to eliminate the infection and minimize any long-term complications [10]. Current treatment of osteomyelitis usually includes a thorough surgical bone debridement and placement of polymethyl metacrylate (PMMA) beads releasing an antibiotic, e.g. gentamicin. However, the resistance developed by the bacteria to antibiotics and the local influence of the treatment have reduced its efficiency [5], nonetheless the research continues in this direction and other antibiotics which are slowly released from carrier matrices have and are being explored [3, 6]. Electrically generated silver ions have been shown to be an antibacterial agent in a broad spectrum of bone and other infections. The beneficial antibacterial effect of silver ions liberated electrically had been proven in treatment of osteomyelitis by different studies. A clinical study involved 920 cases of COM (infected bone wounds) that were treated by wound debridement, silver iontophoresis and proper follow-up wound care, and showed 85% control of bone infection [4]. Other studies reported the successful use of electrically generated silver ions in the management of COM in humans [1, 7] and in animal models [2].

The main drawbacks of the currently used treatments, i.e. surgical debridement, locally applied antibiotics, or electrically liberated silver ions, are their short term effect and restricted local influence. In many cases the infection spreads through the bone where the locally applied treatment does not reach. On the other hand, the prolonged systemic antibiotic treatment which is used in most cases is also not completely efficient, as it is not localized enough. Frequently, after the end of the treatment, infection reappears. Some cases of COM are so resistant to treatment that amputation may be required.

In cases of refractory osteomyelitis a hyperbaric oxygen (HBO) treatment might be used, since oxygen under pressure can assist wound healing [11, 12]. HBO is a relatively safe non-invasive therapy, but it is expensive and requires special hyperbaric chambers. The side effects (middle ear and pulmonary barotraumas and myopia) together with main contraindications (poor cardiac output and severe obstructive pulmonary disease) prevent this method from being widely used for osteomyelitis treatment [13].

The use of silver and silver compounds in medicine has been recorded for thousands of years. Since the late 1800's the use of silver as a bactericide has been documented and studied intensively.

Without going into too many details, representative examples of ways in which silver, and in particular silver ions, are administered to heal or prevent bacterial diseases are:
- topical application; One well known example of the topical application of silver compounds is the use of silver sulfadiazine for the treatment of burns. As another example US 6,923,990 describes stabilized compositions comprised of a silver-thiosulfate ion complex further complexed with an amine. In one embodiment, these compositions are incorporated into wound dressings.
- coating or impregnating medical devices with silver containing materials; Embodiments of US 6,923,990 are concerned with impregnating medical devices such as catheters with the silver-thiosulfate-amine compositions. US 6,663,634 describes a bone screw on which at least part of the surface a thin coating of silver has been deposited. After the screw is installed in a bone, galvanic currents arise near the coated part of the screw, releasing silver ions in that region. Surgical implants comprising small amounts of biodegradable silver are described in US 4,615,705.
- thermocouple junctions; It is a known phenomenon that a localized electric current can be induced to flow when two pieces of dissimilar metals are brought into contact with each other. This phenomenon has been taken advantage of for many years in medicine, specifically in dental medicine. For example US 2,069,112 and US 4,886,075 describe devices comprised of wires of two different metals, for example silver together with either gold or platinum that are twisted or soldered together and inserted into a root canal. As a result of the thermocouple effect, silver ions are released into the canal and surrounding tissue where they heal bacterial infections, help prevent their reoccurrence, and promote bone growth. The device can either be removed from the tooth after a period of time or sealed permanently in the tooth.
- ion generators; Many publications describe methods of producing silver ions around surgical or dental devices in vivo in order to treat bacterial infections and/or to prevent their occurrence. In these devices an electrical circuit comprised of a constant current supply, a first electrode attached in some way to the device, and a second electrode either implanted in the soft tissue near by the first electrode or attached to the device and electrically insulated from the first electrode. The electrolytes in the body fluids complete the circuit when the current supply is activated. One of the electrodes is usually silver and the electric current flowing in the circuit releases silver ions into the body fluids. The silver ions are only released as long as the current source is activated. The principle of the operation of such devices can be understood from, for example US 4, 027,393 and US 4,569,673. A modification of the method is taught in US 4,126,937. In the invention described in this patent, which is a method of treating dental pulp infection, dental cap material comprising silver particles is placed into a cavity prepared in a dental restoration procedure. The dental cap material acts as one electrode, a silver wire is placed into the dental cap material to enable a direct current power source to be connected, a second contact is provided on the patient to complete the circuit and a direct current is applied for a period of about ten minutes. The current source is then removed and the inventor claims that the silver ion activity continues at a decreasing rate for a few hours and indefinitely at a very low level.
- iontophoresis without an external current source; US 5,322,520 teaches an electrode arrangement made up of particles or layers of two different metals that are separated by a resistive material. The electrode arrangement is incorporated into or attached to medical devices, e.g. catheters. When surrounded by and in contact with an electrolytic fluid, i.e. body fluids, galvanic current flows between the two metals and metal ions are released into the fluid.
- The silves electrode temporary electrode and second electrode as defined in claim 1 are known in combination from US 4314 554.

Despite the vast amount of work done in studying the antibacterial effect of silver ions and the many different ways of applying them, there still is no effective, long term, widely accepted method of administrating silver ions for the purpose of treating and preventing bacterial diseases such as osteomyelitis.

It is another purpose of the present invention to provide an apparatus comprising tools and other elements necessary for the treatment of bacterial diseases.

It is another purpose of the present invention to provide an apparatus comprising tools and other elements necessary for the treatment of osteomyelitis.

Further purposes and advantages of this invention will appear as the description proceeds.

### Summary of the Invention The invention is defined in claim 1.

In a first aspect the invention is an apparatus for treatment and prevention of infectious diseases. The apparatus comprises:
a. a trocar;
b. a punch;
c. a pusher;
d. a silver electrode;
e. a temporary electrode;
f. a needle made from a biocompatible material having a negative electrical potential with respect to silver; and
g. one or more second electrodes comprised of the same material as the needle.

The apparatus of the invention may also comprise one or both of an external D.C. voltage source and a conductivity meter.

In different embodiments the apparatus is intended to be used to treat a bacterial infection, which can be either osteomyelitis or chronic osteomyelitis, following an orthopedic procedure or infectious arthritis or a rheumatic malady.

The silver electrode in the apparatus of the invention may be comprised of pure silver or an alloy comprised of pure silver and a small percentage of the material used to make the second electrode. In embodiments of the apparatus the silver electrode weighs between 1mg and 50mg.

The second electrode and the needle in the apparatus of the invention can be made from gold. In embodiments of the apparatus the second electrode weighs between 5mg and 100mg.

The temporary electrode of the apparatus is comprised of a small metallic cylinder surrounded on all but its bottom surface by an electrically insulating layer and connected to a long insulated electricity conducting wire.

An aspect not part of the invention is a method of treatment and prevention of infectious diseases. The method comprises the steps of:
a. boring a hole into a bone or a joint;
b. implanting a small silver electrode into the hole;
c. placing a temporary electrode in the hole, on top of and in intimate contact with the silver electrode;
d. connecting electrically the temporary electrode with a first terminal of a conductivity meter;
e. connecting electrically a needle made from a biocompatible substance having a negative electrical potential with respect to silver to a second terminal of the conductivity meter;
f. touching the skin with the needle at a spot near the hole and measuring the conductivity of the path from the silver electrode to the needle;
g. repeating step f at different locations until the path having the highest conductivity is found and marking the corresponding location of the needle;
h. repeating steps f and g to locate one or more additional corresponding locations, if desired;
i. disconnecting the conductivity meter from the temporary electrode and removing the conductivity meter and the needle;
j. embedding one or more second small electrodes made from the same biocompatible material as the needle subcutaneously at the corresponding locations;
k. connecting electrically one terminal of a D.C. voltage source to the temporary electrode and the second terminal of the D.C. voltage source to a first one of the second electrodes, thereby initiating the release of silver ions by applying a small current between the silver electrode and the first one of the second electrodes;
l. disconnecting the second terminal of the D.C. voltage source from the first one of the second electrodes;
m. repeating steps k and 1 for each of the other second electrodes; and
n. removing the temporary electrode and the D.C. voltage source.

In some embodiments of the method not part of the invention the hole is bored into the infected area of the bone. In other embodiments it is bored in the vicinity of the infected area.

Amongst other diseases, the method not part of the invention can be used to treat a bacterial infection following an orthopedic procedure, e.g. osteomyelitis or chronic osteomyelitis; infectious arthritis; or a rheumatic malady.

According to embodiments of the method not part of the invention the silver electrode is comprised of pure silver or of an alloy comprised of pure silver and a small percentage of the material used to make the second electrode. In embodiments of the method the silver electrode weighs between 1mg and 50mg.

According to embodiments of the method not part of the invention the needle and the second electrode can be made from gold. In embodiments of the invention the second electrode weighs between 5mg and 100mg.

According to embodiments of the method not part of the invention the temporary electrode is comprised of a small metallic cylinder surrounded on all but its bottom surface by an electrically insulating layer and connected to a long insulated electricity conducting wire.

According to embodiments of the method not part of the invention the D.C. voltage source delivers a current of less than 0.5mA to initiate the release of silver ions and the D.C. voltage source delivers a current for between 5 seconds and 120 seconds or more to initiate the release of silver ions.

All the above and other characteristics and advantages of the invention will be further understood through the following illustrative and non-limitative description of preferred embodiments thereof, with reference to the appended drawings.

### Brief Description of the Drawings

- Figs. 1 to 3 schematically show the physical principle of operation of the invention;
- Figs. 4A to 4H schematically show the components of the apparatus of the invention; and
- Figs. 5 to 13 schematically illustrate the steps of the method not part of the invention.

### Detailed Description of Preferred Embodiments

The method not part of the invention for the treatment of bacterial diseases is based on the antibacterial characteristic of the electrically released silver ions. The method not part of the invention overcomes the disadvantages of the local and short term solutions by assuring a long term and spreading effect of the treatment. The method can be used to cure a wide range of diseases, e.g. osteomyelitis, chronic osteomyelitis and infectious arthritis and certain kinds of rheumatic maladies, e.g. rheumatoid arthritis, psoriasis, and lupus. The invention will be illustrated herein in terms of the treatment of chronic osteomyelitis, however the invention is not specifically limited to treatment of this disease and skilled persons will be able to adapt the method and apparatus described herein *mutatis mutandis* for the treatment of other diseases.

The method includes the implantation of a small electrode including silver in the infected bone, embedding another small electrode including another metallic element subcutaneously, and initiating the release of silver ions by applying a small current between the two electrodes. The initiation current is applied for a very short time and then discontinued. After this a continuous release of the silver ions will take place for a long time, which is estimated theoretically to be at least several years, while spreading the silver ions to the surrounding tissue as well. This process assures a long-term antibacterial effect, which after accomplishing the infection healing will also provide preventive care.

The first step is to identify the proper place to install the silver electrode. For the treatment of chronic osteomyelitis the silver electrode should be implanted in the bone within the infected area. The infected location can be determined by standard methods such as x-ray imaging, CT scanning, MRI, etc.

Once the location of the place within the bone at which the silver electrode is to be implanted is determined the bone is prepared using tools contained in the apparatus of the invention. The silver electrode is then implanted, a second electrode is implanted subcutaneously at a location near the silver electrode, and means for applying a low current are connected between the two electrodes, and after a short time disconnected.

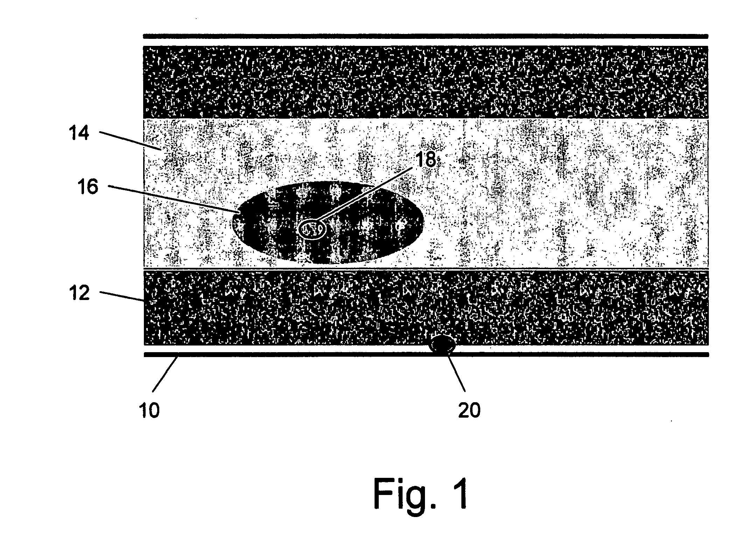

The physical principle of operation of the invention is schematically shown in Figs. 1 to 3. In Fig.1 is shown the skin 10 and muscle 12 layers surrounding bone 14. A silver electrode 18 is shown implanted in infected area 16 of bone 14. A second electrode 20 is shown implanted underneath the skin 10.

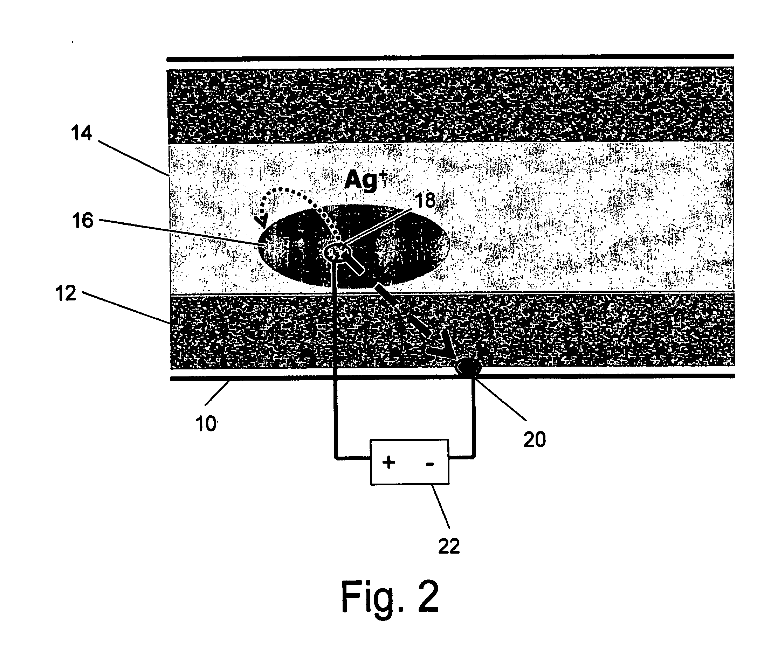

In Fig.2 the positive side of external D.C. voltage source 22 is connected to silver electrode 18 and the negative side of voltage source 22 is connected to the second electrode 20. The bone and muscle tissue conduct electricity and provide a path, shown symbolically by the straight dashed arrow, which completes the electric circuit. The direct current from voltage source 22 cause disintegration of silver electrode 18 releasing silver ions, shown symbolically by the dotted arrow, into the infected area 16 from which they can slowly spread to other areas of the bone, surrounding tissue, and to more remote parts of the body.

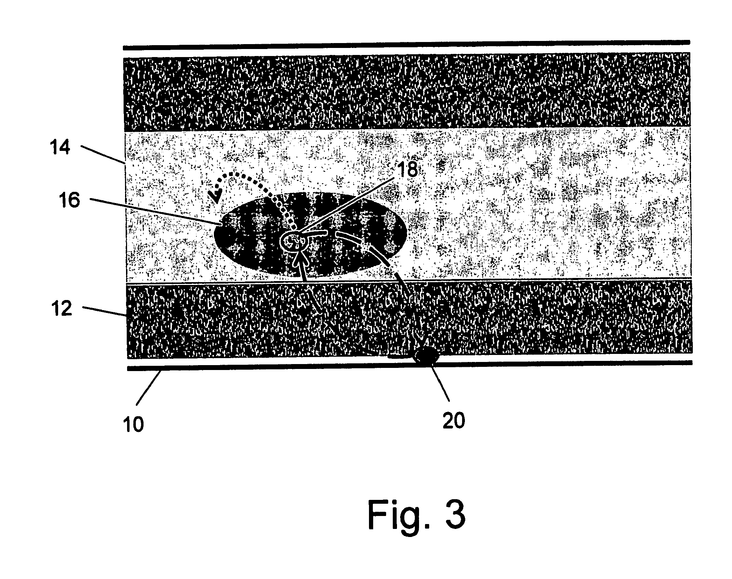

After the initialization of current flow the external D.C. voltage source 22 is disconnected from the electrodes. As shown in Fig.3, the galvanic current continues to flow in what is essentially a galvanic battery comprising silver electrode 18 and second electrode 20 with an electrolyte comprised of the bone and tissue. This current will continue to flow releasing silver ions from silver electrode 18 as long as the two electrodes remain in place, i.e. until one or both of the electrodes 18,20 is either physically removed, totally disintegrates as a result of the prolonged flow of current, or the cathode (the second electrode) becomes completely coated with silver.

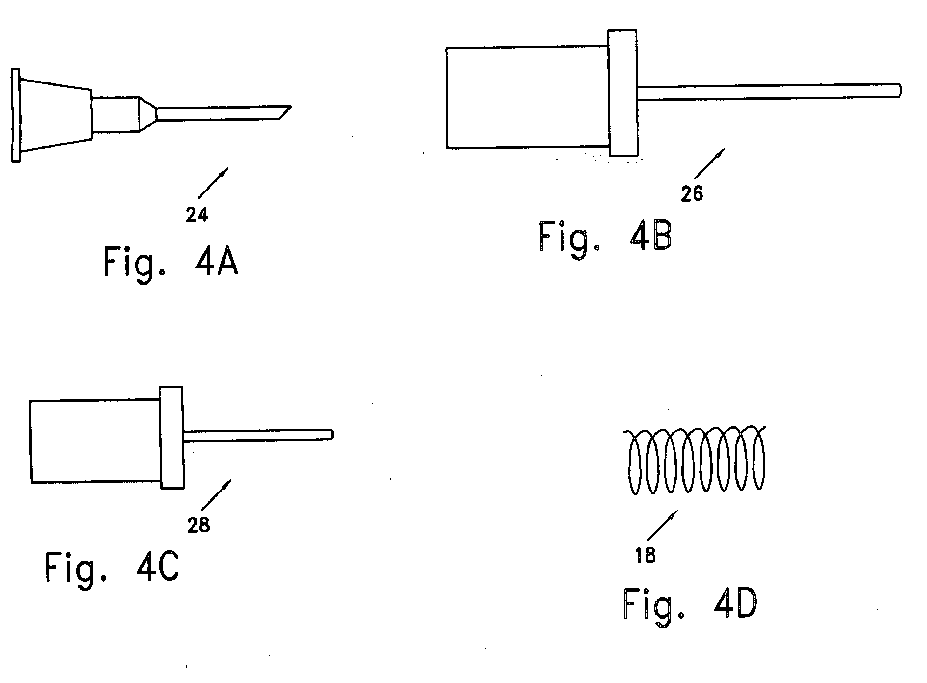

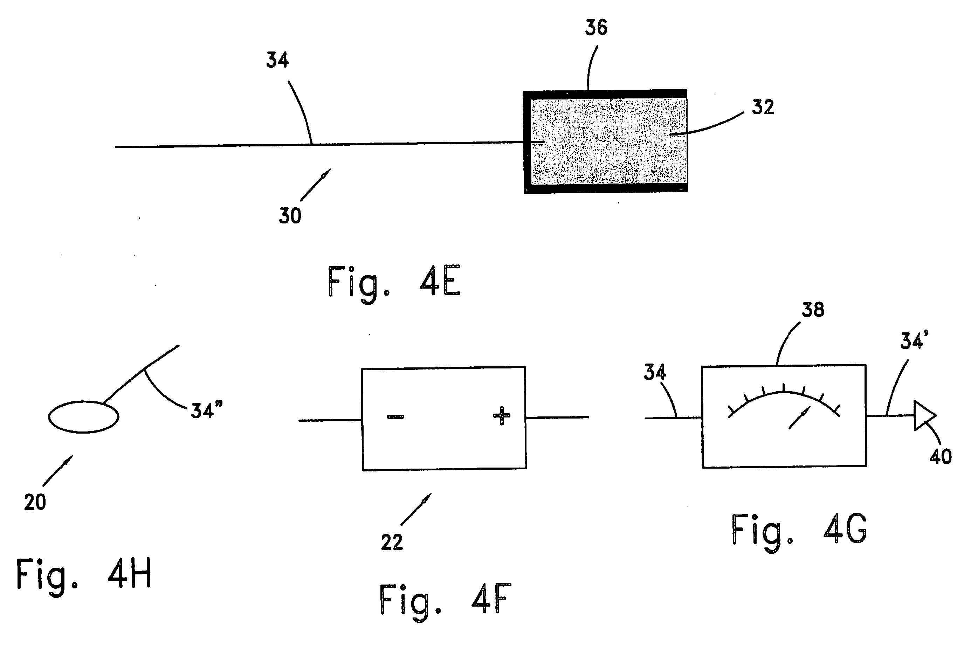

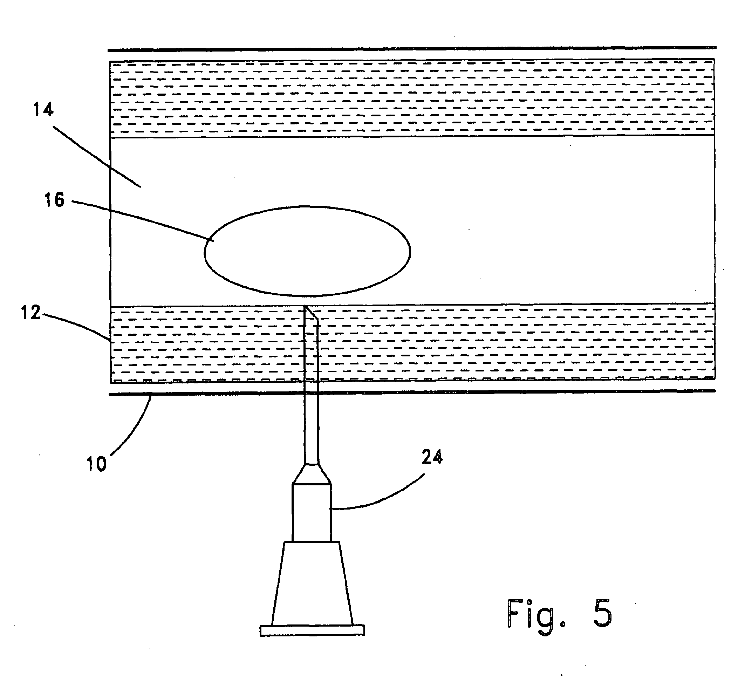

Figs. 4A to 4H schematically show the components of the apparatus of the invention. In these figures: Fig. 4A shows a trocar 24; Fig. 4B shows a punch (or drill) 26; Fig.4C shows a pusher 24; Fig. 4D shows silver electrode 18; Fig. 4E shows temporary electrode 30; Fig. 4F is an external D.C. voltage source 22; Fig. 4G shows conductivity meter 38, with needle 40 connected to it; and 4H is the second electrode 20. Design features and the function of each of these components will be described hereinbelow with reference to Figs. 5 to 13, which schematically illustrate the steps of the method not part of the invention.

To insure sterility, all components of the apparatus are supplied in a "factory-sealed" container, which is opened by the surgeon at the time of performing the procedure. Of course it is also possible that each of the components will be individually wrapped in a sealed "envelope" to maintain its sterility until it is used. It is anticipated that the apparatus will be supplied in two embodiments - a "starter" apparatus, which contains all of the components shown in Figs. 4A to 4H; and a "basic" apparatus which contains all components of the "starter" apparatus with the exception of external D.C. voltage source 22 and conductivity meter 38, which are relatively expensive and clearly reusable. Electrodes 18 and 20 remain inside the body of the patient, but all other components of the basic apparatus are preferably disposed of after a single use.

Fig. 5 shows the first step of the procedure. After using conventional means to locate the infected area 16 inside bone 14 (or joint), trocar 24 is pushed through the skin 10 and layer of muscle 12 contacting the edge of the bone 14 near the center of the infected area. Trocar 24 is a standard trocar used for orthopedic procedures, but it, like other components of the apparatus, can possibly be made to less demanding standards concerning material and strength since it is not meant to be re-sterilized or reused.

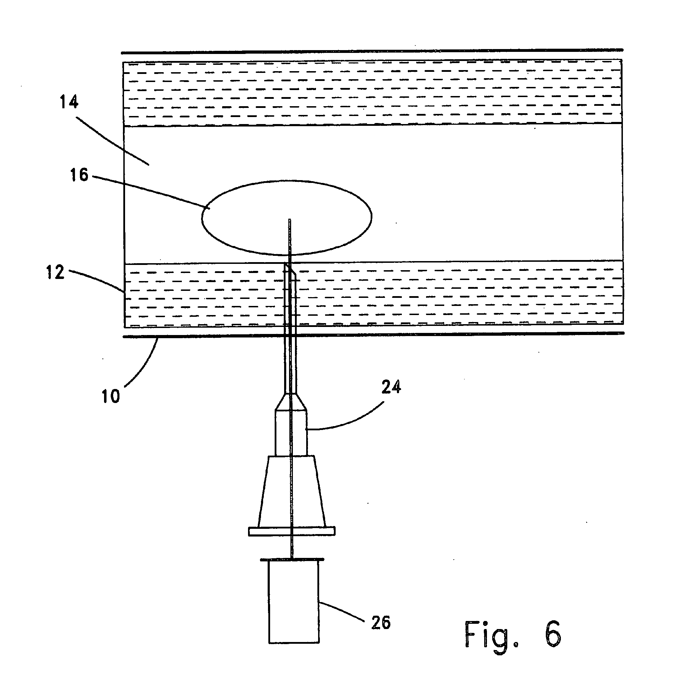

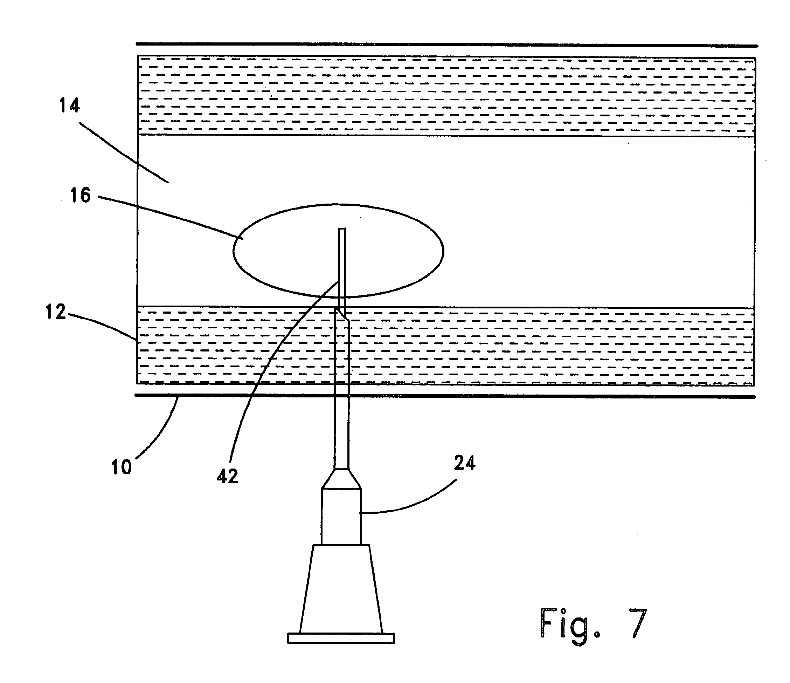

In the step shown in Fig. 6 a hole is made in bone 14 by means of an orthopedic bone punch 26 inserted through trocar 24 and pushed through bone 12 into the infected area 16. In some situations it might be preferable to use a small diameter bone drill instead of a punch. In either case, Fig. 7 shows the situation after the punch/drill 26 has been withdrawn. There remains, at the end of trocar 24, a hole 42 that penetrates the bone and extends into infected area 16.

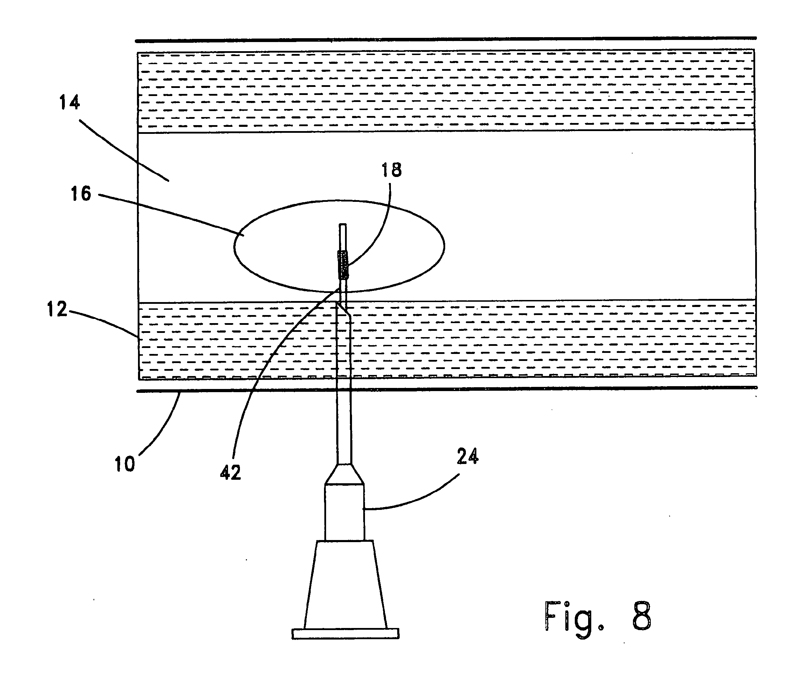

In the next step, the result of which is shown in Fig. 8, pusher 28 (Fig. 4C-not shown in Fig. 8) is used to push silver electrode 18 through trocar 24 to the end of hole 42 that was previously made in bone 14. The pusher is a simple rod or pipe with a plastic handle that can slide through the trocar.

The silver electrode 18 is a small piece of pure silver or a silver containing alloy that typically weighs on the order of 1-50 mg. Silver electrode 18 can be supplied as a small solid piece of metal or preferably is fabricated from silver wire into a small diameter cylindrical coil as shown in Fig. 4D. The hole 42 has a diameter only very slightly larger than the diameter of silver electrode 18 so that the spongy bone tissue inside the hard outer shell of bone 14 will push against silver electrode 18, holding it in place even before the tissue begins to re-grow around it. The effect is even greater, if a punch is used instead of a hole; since, in the former case, the punch tends to push through the spongy tissue to form the hole without removing all of the material as in the later case.

This is an appropriate place to note that none of the figures are drawn to even an approximate scale and that the scale varies from figure to figure. The actual sizes of the various components of the apparatus can be determined by starting with the diameter of silver electrode 18, which is slightly less than the diameter of hole 42, which is equal to the outer diameter of punch 26, etc. It also should be noted that, although the preferred location of the silver electrode for the treatment of osteomyelitis is within the infected area in the bone, in other circumstances other locations in the body in the vicinity of the infected area, e.g. in the metaphysis of a bone might be the preferred location.

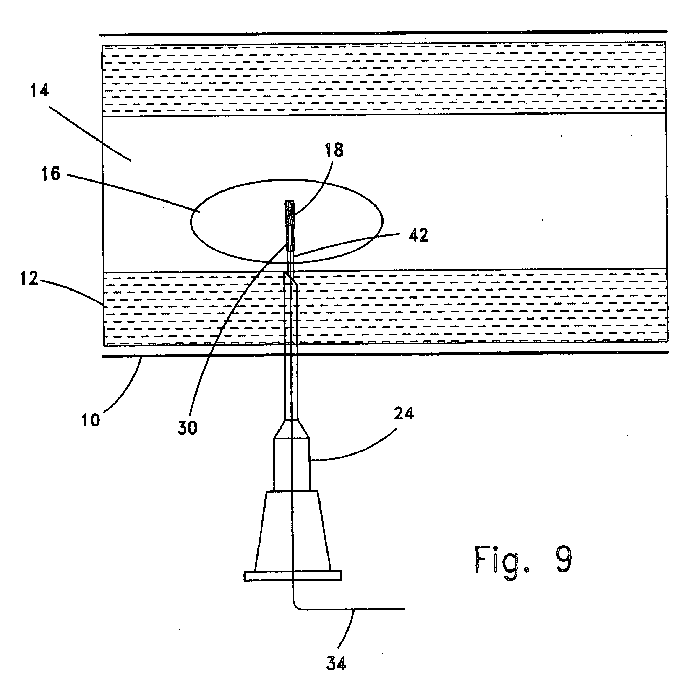

After silver electrode 18 is positioned in hole 42, temporary electrode 30 is inserted into hole 42 on top of silver electrode 18 with the aid of pusher 28. As can be seen in Fig.4E, temporary electrode 30 is comprised of a small metallic cylinder 32 connected to a long insulated electricity conducting wire 34. metal cylinder 32 is surrounded on all but its bottom surface by an electrically insulating layer 36, whose purpose will be explained herein below. As can be seen in Fig. 9, after pusher 28 has been withdrawn, the exposed bottom surface of metallic cylinder 32 of temporary electrode 30 is in contact with the top of silver electrode 18 and the free end of electricity conducting wire 34 extends outside of trocar 24.

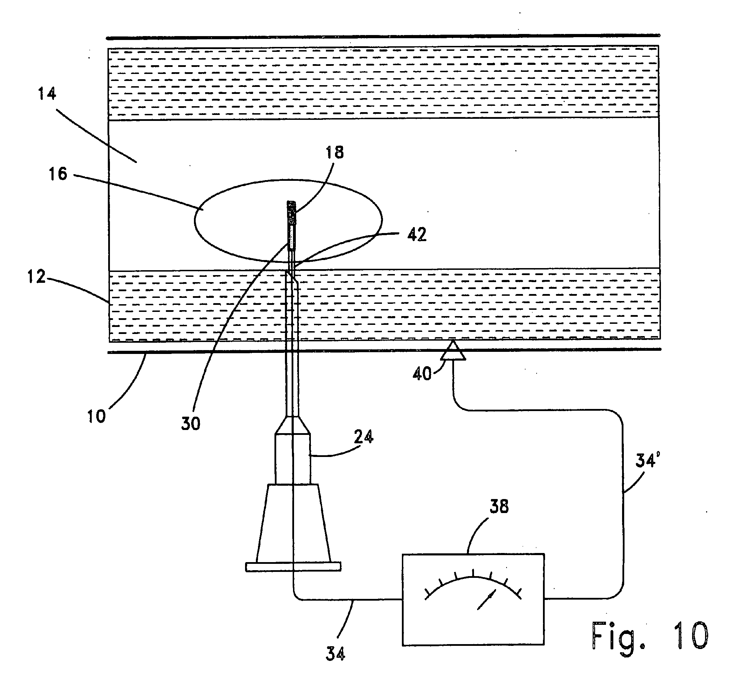

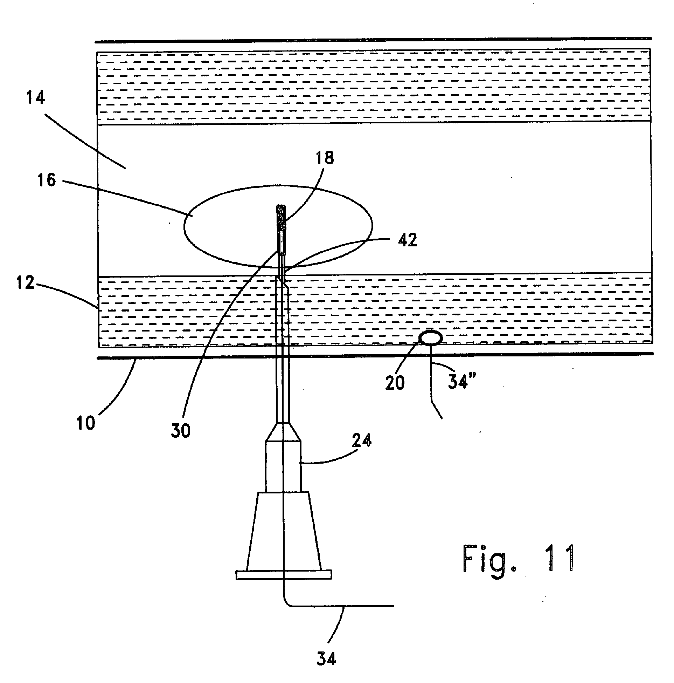

The next step is to determine the location of the second electrode. According to the invention, the most effective results will be obtained if the second electrode is placed at the location under the surface of the skin at which the electrical conductivity between the second electrode and the silver electrode is maximized. Fig. 10 illustrates the method for locating the optimal position for placement of the second electrode. One terminal of conductivity meter 30 is connected to electrically conducting wire 34 of temporary electrode 30, which is in electrical contact with silver electrode 18, embedded in infected area 16 of bone 14. The other terminal of conductivity meter 30 is connected to electrically conducting wire 34', which has a needle 40 attached to its end. Needle 40 is made of the same material as is the second electrode. When needle 40 touches the skin a closed circuit is set up and the meter reads the conductivity of the circuit. If the needle is moved to a different location a different conductivity is read. The difference in conductivity is essentially due to the part of the circuit made up of the electrolyte path (bone and tissue) between the silver electrode 18 and second needle 40. Needle 40 is moved about on the skin surface until the highest value of the conductivity is measured. When this spot is located the conductivity meter is removed and the second electrode 20 is embedded subcutaneously. This is the stage of the procedure that is shown in Fig. 11.

Second electrode 20 is a small, typically 5-100 mg piece of biocompatible material that must have a negative electrical potential with respect to silver. An example of such a material is gold.

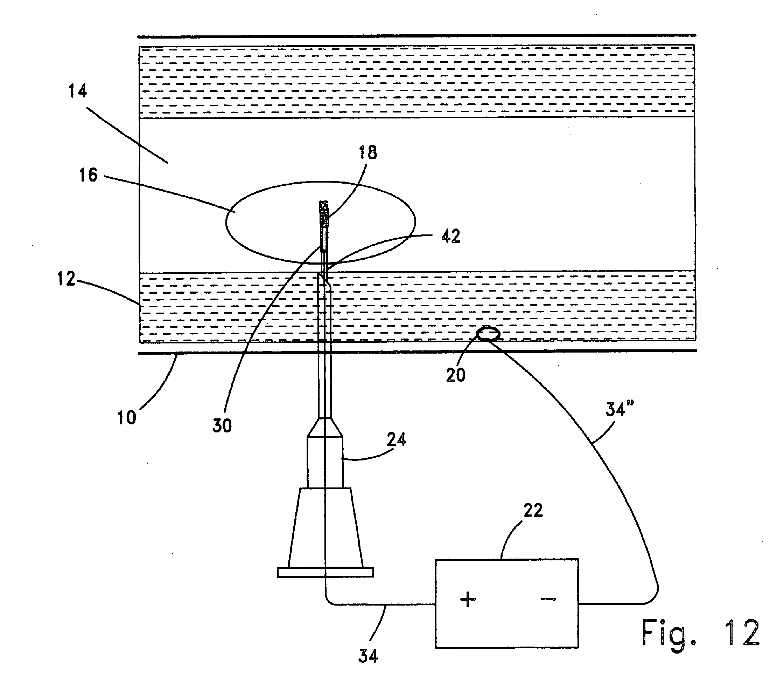

Once both electrodes have been implanted, D.C. voltage source 22 is connected to wire 34 of temporary electrode 30 and to electrically conducting wire 34" connected to second electrode 20 as shown in Fig. 12. When voltage source 22 is activated, a direct current from voltage source 22 begins to flow through the tissue between the two electrodes as shown in Fig. 2 and described hereinabove. It is now apparent why the insulation layer 36 is necessary on temporary electrode 30. If it were not present, then at least some of the current would leak from the sides of the temporary electrode, creating alternative paths to the second electrode.

The starting current is relatively low, on the order of less than 0.5mA and is applied for a short period of time, on the order of 5 to 120 seconds. These parameters of the starting current should be appropriate to start the flow of the current in most situations. It is understood of course that the important parameter is the number of ions released during the start-up period and that this is proportional to the starting current multiplied by the time. Therefore the starting current can be applied for longer or shorter periods of time than those suggested above if the value of the applied electrical current is varied accordingly.

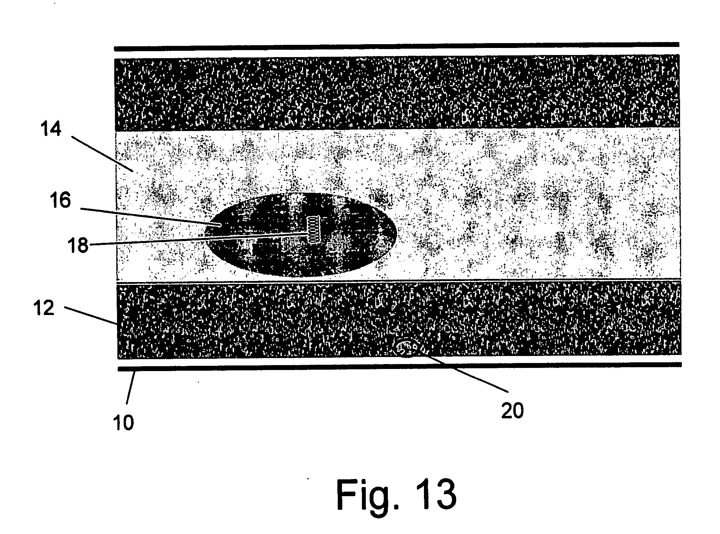

After the original brief starting period, the D.C. voltage source 22, temporary electrode 30, and trocar 24 are removed. Wire 34" is removed from second electrode 20 leaving the situation shown in Fig. 13 at the end of the procedure.

Galvanic currents on the order of a couple of tens of nanoamperes will continue to flow between silver electrode 18 and second electrode 20 for a long period of time. The exact period of time depends on many factors, but the inventor believes that the right conditions can easily be created for the galvanic currents to continue for up to several tens of years. The galvanic current causes a slow but continuous decomposition of the silver electrode. As this electrode erodes, silver ions as well as nano-particles of silver metal are released into the surrounding tissue. The silver ions kill the bacteria in the immediate vicinity of the electrode and gradually drift to other parts inside and eventually outside of the bone and from there to other parts of the body, where they act as a bactericide for any bacteria that they encounter. At the same time the silver nano-particles also move slowly outward from the electrode and are gradually broken down in the body being slowly converted into silver ions.

In another embodiment of the invention the silver electrode is not made of pure silver, but of an alloy of silver with a small percentage of the material used to make the second electrode. In this embodiment, the erosion of the electrode by the galvanic current will result in the release of silver ions and nano-particles of the alloy. As the nano-particles of alloy move around in the body, conditions will arise wherein pairs of them will form nano-size galvanic cells, which will release even more silver ions both locally and eventually in all parts of the body.

In another embodiment of the invention, additional second electrodes can be implanted under the surface of the skin around the silver electrode in the manner describer herein above. This results in the establishment of galvanic currents between the "central" silver electrode and the plurality of second electrodes located around it, thereby increasing the volume of bone and tissue into which the silver ions are released.

The invention has been illustrated in terms of treatment of an existing case of Osteomyelitis, but it could also have an important role in preventing bacterial disease. For example, it is known that Osteomyelitis develops after a significant percentage of orthopedic procedures, sometimes after an extended period when it is felt that the procedure has been a complete success. The cost and complexity of the method not part of the present invention is so small relative to that of most orthopedic procedures and certainly when compared to the long term cost and difficulty of treating a patient who is unfortunate enough to contract Osteomyelitis, that serious consideration should be given to making the use of the invention part of the protocol of many orthopedic procedures by not waiting for infection to set in but by implanting a silver electrode in the bone being treated and carrying out the rest of the steps of the invention as part of the post surgery closing up of the wounds.

The safety and feasibility of the silver ions bone infection treatment of the invention have been demonstrated in-vivo by inducing OM in animals by bacteria inoculation and applying the method not part of the invention to cure them. Rabbits were used for this study as they are a frequently used animal model in OM research [16].

The study had three stages. In the first stage safety was demonstrated, by applying the method not part of the invention to healthy animals in comparison to a control group of healthy and untreated animals. The safety of the method was proven in 100% of the animals by X-Ray, blood tests, gross pathology and histology.

In the second stage the model of inducing well developed bone infection in animals was established, by employing different inoculations.

Finally, in the third stage the feasibility of the method was demonstrated. The right tibia of each of the animals from both the treatment and the control group was inoculated with Staphylococcus aureus [17]. After one week the method not part of the invention was applied to the animals of the treatment group. Half of the animals from both groups were sacrificed at the end of follow-up periods, of 2 and 4 weeks

The X-ray and histology analysis showed that at the end of the follow-up period all of the untreated animals had acute OM, characterized by abscesses formation within the medullary cavity. These findings are indicative that the establishment of the model was successful and self healing did not occur.

In the majority of the treated animals in the long term follow-up group, an apparent reduction in the size of the intramedullary abscess was noted. In one animal complete healing was observed. In the animals of the short term follow-up, the start of a healing process has been observed in about half of the animals.

At this stage additional safety evidence was provided by blood tests, gross pathology and histological analysis performed in all animals, to demonstrate that the quantities and nature of the elements used (silver and complementary) did not affect the general health of the animals.

Although embodiments of the invention have been described by way of illustration, it will be understood that the invention may be carried out with many variations, modifications, and adaptations, without exceeding the scope of the claims.

### Bibliography

1. Becker R. O., Spadaro J. A., Treatment of orthopaedic infections with electrically generated silver ions. A preliminary report. J. Bone Joint Surg. Ann. 1978, 60(7):871-81.
2. Ducland R., Spadaro J. A., Rahn B. A., Silver antibacterial bone cement. Comparison with gentamicin in experimental osteomyelitis. Clin. Orthop. Relat. Res. 1982, 169:264-8.
3. Faber C., Stallman H.P. Lyanau D. M., et. al., Release of antimicrobial peptide Dhvar-5 from polymethylmethacrylate beads. Journal of Antimicrobial Chemotherapy 2003, 51:1359-64.
4. Nand S., Sengar G. K., Nand S., et. al., Dual use of silver for management of chronic bone infections and infected non-unions. J. Indian Med. Assoc. 1996, 94(3):91-5.
5. Stallman H.P., Faber C., Bronckers L.J.J., et. al., Osteomyelitis prevention in rabbits using antimicrobial peptide hLF1-11 or gentamicin-containing calcium phosphate cement. Journal of Antimicrobial Chemotherapy 2004, 54:472-6.
6. Vogely H. C., Oosterbos C. J., Puts E. W. et. al., Effects of hydroxyapatite coating on Ti-6A1-4V implant-site infection in a rabbit tibial model. Journal of Orthopaedic Research 2000, 18:485-93.
7. Webster D. A., Spandara J. A., Becker R. O., Krammer S., Silver anode treatment of chronic osteomyelitis. Clin. Orthop. Relat. Res. 1981, 161:105-14.
8. Zuluaga A. F., Galvis W., Jaimes F. and Vesga O., Lack of microbiological concordance between bone and non-bone specimens in chronic osteomyelitis: an observational study. BMC Infectious Diseases, 2002, 2:8.
9. Osteomyelitis, Copyright 1995-2005, The Cleveland Clinic Foundation, http://www.clevelandclinic.org/health/health-info/docs/2700/2702.asp?index=9495&src=news.
10. Osteomyelitis, Robert Wood Johnson University Hospital, http://www.rwjuh.edu/health information/adult bone osteom.html
11. Hunt TK, Linsey M, Grislis HJ, et al. The effect of differing ambient oxygen tensions on wound infection. Ann Surg 1975; 181: 35-9.
12. Mendel V, Reichert B, Simanowski HJ, Scholz HC. Therapy with hyperbaric oxygen and cefazolin for experimental osteomyelitis due to Staphylococcus aureus in rats. Undersea Hyperb Med 1999; 26(3): 169-74.
13. Wright J., Hyperbaric oxygen therapy for wound healing, World Wide Wounds, May 2001, http://www.worldwidewounds.com/2001/april/Wright/HyperbaricOxyg en.html
14. Lazzarini L., et. al., Osteomyelitis in long bones, J. Bone Joint Surg. Am. 86:2305-2318, 2004.
15. McCarthy J, et. al., Muskoskeletal infections in children. basic treatment principles and recent advancements, J. Bone Joint Surg. Am. 86:850-863, 2004.
16. Dekel S. and M. J. 0. Francis, The treatment of Osteomyelitis of the Tibia with Sodium salicylate. An experimental study in rabbits, JBJS, VOL. 63-B, No. 2. 981.
17. Stallman H.P., Faber C., Bronckers L.J.J., et. al., Osteomyelitis prevention in rabbits using antimicrobial peptide hLF1-11 or gentamicin-containing calcium phosphate cement. Journal of Antimicrobial Chemotherapy 2004, 54:472-6.

## Claims

1. An apparatus for treatment and prevention of infectious diseases in bone tissue surrounded by at least a portion of skin and muscle tissue, the apparatus comprising:
a. a trocar (24) for insertion through the skin and muscle tissue ;
b. a punch (26) for insertion through said trocar, to create an opening in the bone tissue;
c. a pusher (28);
d. a silver electrode (18) for entering said opening in the bone tissue through said trocar by being pushed through said trocar by said pusher;
e. a temporary electrode (30) for entering said opening in the bone tissue through said trocar by being pushed through said trocar by said pusher on top of said silver electrode;
f. a needle (40) made from a biocompatible substance having a negative electrical potential with respect to silver;
g. one or more second electrodes (20) made from same material as said needle for insertion through the skin and muscle tissue at a location separated from said opening in the skin and muscle tissue, and
h. a conductivity meter (38) attached to said needle, wherein upon contacting said needle to the skin tissue at different locations, location with highest conductivity reading determines said location for said one or more second electrodes.

2. An apparatus according to claim 1, comprising an external D. C. voltage source adapted to cause an initial current to flow between said silver electrode and said one or more second electrodes through one or more connective wires.

3. An apparatus according to claim 1, wherein said silver electrode comprises pure silver.

4. An apparatus according to claim 1, wherein said silver electrode comprises a combination of pure silver and a small percentage of the material used to make said second electrode.

5. An apparatus according to claim 1, wherein said silver electrode weighs between 1mg and 50mg.

6. An apparatus according to claim 1, wherein said needle and said second electrode are made from gold.

7. An apparatus according to claim 1, wherein said second electrode weighs between 5mg and 100mg.

8. An apparatus according to claim 1, wherein said temporary electrode comprises a small metallic cylinder surrounded on all but its bottom surface by an electrically insulating layer and connected to a long insulated electricity conducting wire.

9. An apparatus according to claim 2, wherein said D.C. voltage source is configured to delivery a current of less than 0.5mA to initiate release of silver ions.

10. An apparatus according to claim 9, wherein said D.C. voltage source is configured to deliver a current for between 5 seconds and 120 seconds to initiate said release of silver ions.

11. An apparatus according to claim 1, wherein said punch is for pushing through the bone tissue into an infected area thereof.

12. An apparatus according to claim 1, wherein said silver electrode is fabricated from silver wire into a cylindrical coil.

## Patentansprüche

1. Ein Apparat zur Behandlung und Prävention von Infektionskrankheiten im Knochengewebe, das zumindest von einem Teil von Haut- und Muskelgewebe umgeben ist, wobei der Apparat Folgendes umfasst:
a. einen Trokar (24) zum Einführen durch Haut- oder Muskelgewebe;
b. eine Stanze (26) zum Einführen durch den Trokar, um eine Öffnung im Knochengewebe zu machen;
c. einen Schieber (28),
d. eine Silberelektrode (18), zum Einbringen in die Öffnung im Knochengewebe durch den Trokar, indem sie mit dem Schieber durch den Trokar geschoben wird;
e. eine temporäre Elektrode (30) zum Einbringen in die Öffnung im Knochengewebe durch den Trokar, indem sie oben auf der Silberelektrode mit dem Schieber durch den Trokar geschoben wird;
f. eine Nadel (40), die aus einer biokompatiblen Substanz hergestellt wurde, welche ein negatives elektrisches Potenzial im Hinblick auf Silber aufweist;
g. eine oder mehrere zweite Elektroden (20) aus demselben Material wie die Nadel zum Einführen durch das Haut- und Muskelgewebe an einer Stelle, die von der Öffnung im Haut- und Muskelgewebe getrennt ist, und
h. ein an der Nadel befestigtes Leitfähigkeitsmessgerät (38), worin, nachdem die Nadel an verschiedenen Stellen mit der Haut in Kontakt gekommen ist, über die Stelle mit dem höchsten Leitfähigkeitsmesswert die Stelle für die eine oder mehreren zweiten Elektroden bestimmt wird.

2. Ein Apparat gemäß Anspruch 1, der eine externe Gleichspannungsquelle beinhaltet, welche adaptiert wurde, um den initialen Stromfluss zwischen der Silberelektrode und der einen oder mehreren zweiten Elektroden durch einen oder mehrere Verbindungsdrähte auszulösen.

3. Ein Apparat gemäß Anspruch 1, worin die Silberelektrode aus reinem Silber besteht.

4. Ein Apparat gemäß Anspruch 1, worin die Silberelektrode aus einer Kombination aus reinem Silber und einem geringen Prozentsatz des für die Herstellung der zweiten Elektrode verwendeten Materials besteht.

5. Ein Apparat gemäß Anspruch 1, worin die Silberelektrode zwischen 1 mg und 50mg wiegt.

6. Ein Apparat gemäß Anspruch 1, worin die Nadel und die zweite Elektrode aus Gold sind.

7. Ein Apparat gemäß Anspruch 1, worin die Elektrode zwischen 5mg und 100mg wiegt.

8. Ein Apparat gemäß Anspruch 1, worin die temporäre Elektrode einen kleinen metallischen Zylinder umfasst, der außer an der Unterfläche von einer elektrischen Isolierschicht umgeben ist und mit einem langen, isolierten stromleitenden Draht verbunden ist.

9. Ein Apparat gemäß Anspruch 2, worin die Gleichstromquelle so konfiguriert ist, dass ein Strom von weniger als 0,5mA geliefert wird, um die Freisetzung von Silberionen zu veranlassen.

10. Ein Apparat gemäß Anspruch 9, worin die Gleichstromquelle so konfiguriert ist, dass sie 5 bis 120 Sekunden lang Strom liefert, um die Freisetzung von Silberionen zu veranlassen.

11. Ein Apparat gemäß Anspruch 1, worin die Stanze dazu dient, durch das Knochengewebe in einen infizierten Bereich davon geschoben zu werden.

12. Ein Apparat gemäß Anspruch 1, worin die Silberelektrode aus Silberdraht zu einer zylindrischen Spule verarbeitet wird.

## Revendications

1. Appareil pour le traitement et la prévention des maladies infectieuses dans le tissu osseux entouré par au moins une partie de peau et de tissu musculaire, l'appareil comprenant :
a. un trocart (24) pour l'insertion à travers la peau et le tissu musculaire ;
b. un emporte-pièce (26) pour insertion à travers ledit trocart, pour créer une ouverture dans le tissu osseux ;
c. un poussoir (28),
d. une électrode d'argent (18) pour entrer ladite ouverture dans le tissu osseux à travers ledit trocart en étant poussé à travers ledit trocart par ledit poussoir ;
e. une électrode temporaire (30) pour entrer ladite ouverture dans le tissu osseux à travers ledit trocart en étant poussé à travers ledit trocart par ledit poussoir au-dessus de ladite électrode en argent ;
f. une aiguille (40) fabriquée à partir d'une substance biocompatible ayant un potentiel électrique négatif par rapport à l'argent ;
g. une ou plusieurs deuxièmes électrodes (20) en matériau identique à ladite aiguille pour une insertion à travers la peau et le tissu musculaire à un emplacement séparé de ladite ouverture dans la peau et le tissu musculaire, et
h. un conductivimètre (38) fixé à ladite aiguille, dans lequel lors du contact de ladite aiguille avec le tissu de la peau à des emplacements différents, l'emplacement à la lecture la plus élevée de conductivité détermine ledit emplacement pour ladite ou lesdites secondes électrodes.

2. Dispositif selon la revendication 1, comprenant une source de tension continue externe adaptée pour entraîner le flux d'un courant initial entre ladite électrode d'argent et ladite ou lesdites deuxièmes électrodes à travers un ou plusieurs fils conjonctifs.

3. Dispositif selon la revendication 1, dans lequel ladite électrode d'argent comprend de l'argent pur.

4. Dispositif selon la revendication 1, dans lequel ladite électrode d'argent comprend une combinaison d'argent pur et un petit pourcentage du matériau utilisé pour fabriquer ladite seconde électrode.

5. Dispositif selon la revendication 1, dans lequel ladite électrode d'argent pèse entre 1 mg et 50 mg.

6. Dispositif selon la revendication 1, dans lequel ladite aiguille et ladite seconde électrode sont fabriquées à partir d'or.

7. Dispositif selon la revendication 1, dans lequel ladite deuxième electrode pèse entre 5 mg et 100 mg.

8. Dispositif selon la revendication 1, dans lequel ladite électrode temporaire comprend un petit cylindre métallique entouré sur toute sa surface, sauf sur sa surface inférieure, par une couche électriquement isolante et relié à un long fil conducteur d'électricité isolé.

9. Dispositif selon la revendication 2, dans lequel ladite source de tension continue est configurée pour délivrer un courant de moins de 0,5 mA pour entreprendre la libération d'ions argent.

10. Dispositif selon la revendication 9, dans lequel ladite source de tension continue est configurée pour délivrer un courant de entre 5 secondes et 120 secondes pour entreprendre ladite libération d'ions argent.

11. Dispositif selon la revendication 1, dans lequel ledit emporte-pièce est destiné à pousser à travers le tissu osseux dans une zone infectée de celui-ci.

12. Dispositif selon la revendication 1, dans lequel ladite électrode d'argent est fabriquée à partir d'un fil d'argent sur une bobine cylindrique.
